# EUROPEAN PATENT APPLICATION

(11) **EP 1 767 182 A1**
(43) Date of publication of application: **28.03.2007**
(21) Application number: 05749014.6
(22) Date of filing: 09.06.2005
(51) Int. Cl.: A61J 15/00, A61M 25/00

(54) **MEMBER FOR CONFIRMING POSITION OF CATHETER IN BODY AND CATHETER ENABLING CONFIRMATION OF ITS POSITION IN BODY**

(30) Priority: 10.06.2004 JP 2004173188
(71) Applicant: JMS Co., Ltd., Hiroshima-shi, Hiroshima 730-8652 (JP)
(72) Inventor: FUJISHIMA, Ichiro, Seireimikatahara Hosp. R. Ctr., Hamamatu-shi, Shizuoka 433-8558 (JP); TOYOTA, Koichiro, JMS.CO., LTD, Hiroshima-shi, Hiroshima 730-8652 (JP); KAWARABATA, Shigeki, JMS.CO., LTD, Hiroshima-shi, Hiroshima 730-8652 (JP); FUZII, Junya, JMS.CO., LTD, Hiroshima-shi, Hiroshima 730-8652 (JP); SATOU, Yoshinori, JMS.CO., LTD, Hiroshima-shi, Hiroshima 730-8652 (JP)
(74) Representative: Röthinger, Rainer
(86) International application number: PCT/JP2005/010566
(87) International publication number: WO 2005/120434

(57) **Abstract**

The present invention provides a member for confirming the position of a catheter placed in a body. The member is **characterized by** including a sound generating unit, and **characterized in that** a sound generated from the sound generating unit can propagate through the catheter. The present invention also provides a catheter attached with the member for confirming the position of the catheter. It is therefore possible to confirm the position of the catheter inserted in the body more easily, reliably, and safely, and also independently of the state inside the stomach of a patient. Accordingly, the present invention was able to provide the catheter which solves problems of the conventional techniques.

## Description

### Technical Field

The present invention relates to a member for confirming the existence position of a catheter in a body, a catheter including the member for the confirmation, and a method of confirming the leading end position or the entire location position of a catheter placed in a body for which the catheter including the members for confirming the position of the catheter is used.

### Background Art

In placing a catheter in a body, to accurately and easily locate the existence position, particularly the leading end position, of the catheter in the body is an important issue to be solved in the field of medical technology. In particular, in the case of a nutrition catheter placed in a digestive tract such as the stomach and the intestine to provide a patient with a nutritional agent, to accurately confirm the leading end position of the catheter is very important. This is because, if the nutritional agent is provided with the catheter wrongly placed in a trachea or a lung, the patient may develop pneumonia and may be exposed to the risk of losing his life in some cases. To solve this problem, a variety of technical means have been proposed. As one of the means, the following method of confirming the position of a catheter has been employed in placing the catheter in the stomach tube or the like.

Confirmation is made on whether the gastric fluid can be suctioned from the catheter placed in a body (in some cases, confirmation is further made with a litmus paper on whether the fluid is acidic to ensure that the fluid is the gastric fluid), and air is blown in through the catheter to confirm whether a foaming sound is generated. Further, if necessary, the position of the catheter is ascertained by using X-ray photography. However, there are cases in which this method cannot be employed due to the accumulation state of the gastric fluid of a patient. Further, the reduction in QOL of the patient, particularly the infant or pediatric patient, which is caused by the radiation exposure by the X-ray photography, has been a very grave problem.

Further, a method has been proposed in which a radiation-emitting radioactive substance is disposed at the leading end position of a catheter placed in a body and the radiation is detected by an extracorporeal detector to thereby detect and ascertain the leading end position of the catheter (Patent Document 1). In this method, however, a radiation-emitting metal is used. Therefore, the method has the problem of causing an adverse effect on the health of the patient and an operator.

Furthermore, a method of confirming the leading end position of a catheter has been proposed which combines an apparatus including a permanent magnet disposed at the leading end thereof for detecting the leading end position of a catheter including the permanent magnet disposed at the leading end thereof (Patent Document 2) with a special catheter (Patent Document 3). In this method of confirming the leading end position, however, the special catheter is used. Thus, a conventionally usually used catheter cannot be used. Further, a special detector needs to be used to confirm the leading end position of the catheter. As a result, the method a problem in that the cost for implementing the method is increased.
Patent Document 1: United States Patent No. 5099845
Patent Document 2: PCT Japanese Translation Patent Publication No. 9-503054
Patent Document 3: PCT Japanese Translation Patent
Publication No. 2000-512873

### Disclosure of the Invention

### Problem to be Solved by the Invention

The problem to be solved by the present invention is to provide a catheter which solves the problems of the above conventional techniques and which can more easily, reliably, and safely confirm the existence position of a catheter placed in a body, and to provide a member used in the catheter for confirming the position of the catheter.

### Means to Solve the Problem

The first aspect of the present invention provides a member for confirming the existence position of a catheter in a body. The member is characterized by including a sound generating unit, and is characterized in that a sound generated from the sound generating unit can propagate through the catheter. Accordingly, the above problem could be solved.
The member for confirming the position of the catheter may include a sound transmitting unit, in addition to the sound generating unit. The members for confirming the position of the catheter are characterized in that the sound generated from the sound generating unit can propagate through the catheter immediately from an opening of the sound generating unit or via the sound transmitting unit and an opening formed at the leading end side of the transmitting unit.
The sound transmitting unit includes a straight pipe or a branch pipe including two or more branch portions. If the sound transmitting unit is a branch pipe including two branched paths, for example, one of the branched paths is connected to the sound generating unit, while the other one of the branched paths is connected to a container of a drug solution to be administered into the body.

The sound generating unit includes the one in which the sound is produced by airflow, such as a member having the structure of a pipe including a recorder pipe and a reed pipe, for example. As the sound generating unit in which the sound is produced by airflow, a cylindrical vent member including a valve structure, particularly a check valve, is preferable. The sound caused by the airflow can be produced as the airflow is blown into the sound generating unit by an airflow blowing member, such as a syringe, a balloon, or a dropper, for example. However, the sound generating unit is not limited to the one in which the sound is produced by airflow, but may be the one which itself produces the sound, such as a member generating an electronic sound, for example. Further, the sound generated in the sound generating unit preferably has a sound volume and/or frequency controlled to be easily detected from outside the body by the sound means, such as a stethoscope, for example.

If the vent member in which the sound is produced by airflow and which includes the valve structure, particularly the vent member including the check valve as the valve structure, is used as the sound generating unit, the sound generating unit exerts, in addition to the effect of producing a sound, the effect of preventing the body fluid from being discharged from inside the body through the catheter to the outside of the body, including the effect of preventing the stomach fluid from being discharged outside the body due to the reflux of the fluid, for example. Further, if the sound generating unit in which the sound is produced by airflow is used, and even if the sound generating unit does not include the valve structure, the sound generating unit similarly can exert the effect of preventing the body fluid, such as the stomach fluid, for example, from being discharged outside the body through the catheter due to the reflux of the fluid, during the time in which the airflow is blown in.

In particular, it is preferable to use two or more of the sound generating units each formed by the vent member including the valve structure, with the sound generating units connected in series with each other, since the sound can be more reliably produced than in the case in which only the single sound generating unit is used. That is, if the sound generating unit is the vent member including the valve structure, and if the sound producing ability or the sound volume of the vent member is too insufficient for the sound detecting means provided outside the body to confirm the position of the catheter placed in the body, the sound generating unit cannot be used. However, even if the sound generating unit has such an insufficient sound producing ability or sound volume, a sufficient sound producing ability or sound volume can be provided by using in combination two or more of the sound generating units.
The phenomenon described above is presumed to be due to the resonance phenomenon occurring between the vent members each including the valve structure or due to the change in the flow velocity or the pressure of flowing gas. In the catheter according to the present invention including the sound generating units, the above phenomenon is utilized, and two or more of the sound generating units are connected in series with each other to be used. Accordingly, the catheter can exert an excellent effect in that the catheter can be immediately used as a product, without previously going through a troublesome test in the manufacturing process of the catheter to determine whether the sound generating units have the sufficient sound producing ability or sound volume required to confirm the position of the catheter placed in the body.

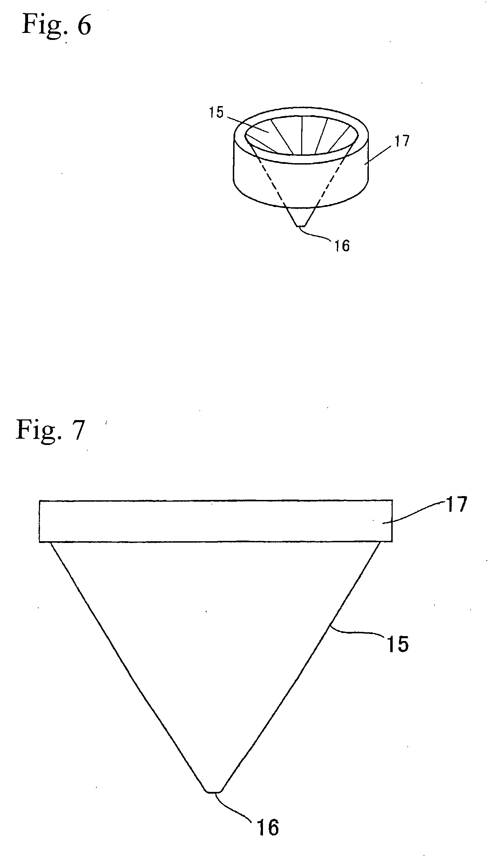

The vent member including the valve structure includes, for example, the medical check valve described in Japanese Unexamined Patent Application Publication No. 10-118178 proposed by the present applicant, which is illustrated in Figs. 6 and 7. The check valve is characterized by including a sloped portion 15 including a front surface having a concave portion depressed in a mortar shape toward the center thereof from a cylindrical portion 17 and a back surface having a convex portion projecting toward the center thereof in the same direction as that of the depression formed in the front surface, an insertion hole 16 formed in the sloped portion and passing through at least the lowest point of the concave portion, and a peripheral wall formed around the sloped portion. The cylindrical portion 17 and the sloped portion of the check valve include the ones formed of an elastic material, such as a silicone, a natural rubber, a synthetic rubber, or a thermoplastic elastomer, for example. Further, it is preferable to integrally mold the respective components.
The vent member of the present invention including the valve structure is not limited to the one described in Japanese Unexamined Patent Application Publication No. 10-118178. Thus, the type or the configuration of the vent member is not limited, as long as the vent member can form the sound generating unit, and more preferably as long as the vent member can prevent fluid from flowing from inside the body toward outside the body.

The above-described member for confirming the existence position of the catheter in the body, which includes the sound generating unit or which includes the sound transmitting unit in addition to the sound generating unit, can be connected to an end portion of the catheter exposed to the outside of the body, i.e., the end portion opposite to an end portion of the catheter placed on the biological body side in the body. Therefore, the member has advantages in that
(1) a variety of sound generating members can be used as the sound generating unit,
(2) as the sound generated in the sound generating unit is caused to propagate through the catheter placed in the body, the entire position as well as the leading end position of the catheter can be easily confirmed by the sound detecting means provided outside the body, and
(3) there is no need to use a catheter of a special structure as the above catheter.

In some cases, however, the sound generating unit leaks a sound therefrom, and the sound leaking from the sound generating unit becomes an obstacle to the confirmation of the leading end position and/or the entire position of the catheter by the sound detecting means provided outside the body. It is therefore preferable to eliminate or at least reduce the sound leaking from the sound generating unit. To solve this problem, it is effective to couple the airflow blowing member to the sound generating unit via the interposition of a tube having a smaller inner diameter than the inner diameters of members existing at the biological body side from the sound generating unit. To solve the above problem, it is also effective to employ such means as attaching a soundproof member to the sound generating unit or covering the sound generating unit with the soundproof member, or to form the sound generating unit itself from, for example, a resin having high ability to prevent sound leakage.

The second aspect of the present invention provides a catheter placed in a body. The catheter is characterized by including a sound generating unit disposed at a leading end portion thereof placed in the body. Accordingly, the above-described problems of the conventional techniques in confirming the existence position of a catheter in a body could be solved.
The catheter includes the sound generating unit at the leading end portion thereof placed in the body. Accordingly, the catheter has the advantage of being capable of allowing the sound detecting means provided outside the body to more accurately confirm the leading end position of the catheter than the above-described catheter in which the sound generating unit is provided outside the body.

The sound generating unit provided at the leading end portion of the catheter placed in the body includes the sound generating unit formed by processing the leading end itself of the catheter. Such a sound generating unit includes, for example, the one obtained by forming a small hole in the leading end portion of the catheter and forming the catheter itself into a shape similar to that of a recorder pipe, the one obtained by crushing the leading end portion of the catheter into a flat plate shape and making an incision in the leading end portion, or the one obtained by providing the leading end portion of the catheter with a valve, particularly a check valve. Further, the sound generating unit may be the one obtained by providing the leading end portion of the catheter with a ready-made pipe, such as a small reed pipe, for example. In these sound generating units provided at the leading end portion of the catheter placed in the body, a sound can be produced by blowing airflow into the units, for example. Further, a member similar to the above-described airflow blowing member can be used as a member for blowing the airflow.
Furthermore, the sound generating unit may be the one obtained by attaching a minute member for generating an electronic sound to the leading end portion of the catheter or by burying the member in the leading end portion.
In the present invention, the leading end portion of the catheter refers to the leading end itself of the catheter and the vicinity of the leading end itself of the catheter.

In the catheter according to the present embodiment, if the sound generating unit includes the check valve similarly to the sound generating unit or the catheter according to the above first aspect of the present invention, the catheter can exert the effect of capable of preventing the stomach fluid from being discharged outside the body through the catheter, in addition to the effect of producing a sound. Similarly, if the catheter uses the airflow blowing means but does not use the sound generating unit including the check valve, the catheter can similarly exert the effect of capable of preventing the stomach fluid from being discharged outside the body through the catheter during the time in which the airflow is blown in.

In placing the catheter including the sound generating unit according to the present invention in the body and in confirming with the sound detecting means provided outside the body the position of the catheter in the body, which includes the leading end position of the catheter and the entire position of the catheter including the leading end position, the conventional techniques described in the above section of Background Art may be used in combination. Further, to confirm each of the leading end position and the entire position, it is preferable to employ the sound generation unit which generates a sound suitable for confirming the position. For example, the position can be confirmed by using, as the sound generating unit, a member which generates an electronic sound, and by using an electronic sound having a frequency suitable for emitting a sound for the leading end position or the entire position. Furthermore, the leading end position or the entire position of the catheter can be confirmed by the sound volume of the sound emitted by the sound generating unit. For example, the position can be confirmed also by adopting an appropriate sound volume for confirming the leading end position or the entire position of the catheter.

In the present specification, a nutrition catheter is specifically described as the catheter including the sound generating unit according to the present invention. However, as well as the nutrition catheter, the catheter including the sound generating unit according to the present invention includes within the range thereof a catheter which is placed in a body and the entire location or the leading end position of which needs to be confirmed by sound detecting means provided outside the body, such as a catheter including a test unit and/or a treatment unit, for example. However, if the catheter including the sound generating unit according to the present invention is the one in which a sound is generated by blowing airflow into the sound generating unit, and if the airflow emitted from the sound generating unit is expected to leak into the biological body, the field of application of the catheter is limited to the treatment field in which introduction of the airflow is biologically accepted.

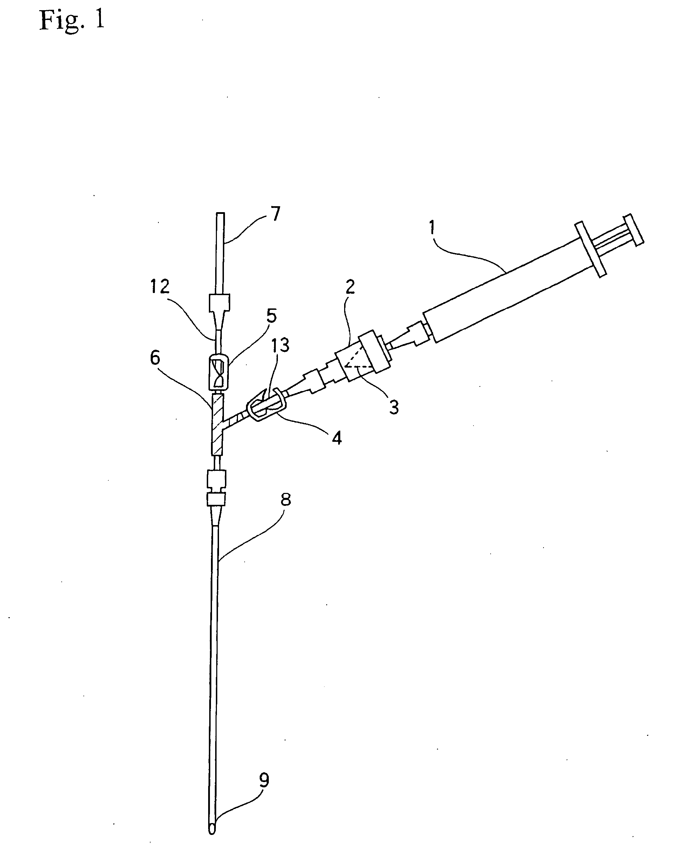

The present invention will be specifically described below on the basis of Fig. 1. Fig. 1 illustrates a syringe 1 which is the airflow blowing member, a sound generating unit (a vent member including a check valve 3) 2 in which a sound is produced by airflow blown therein by the syringe 1, a clamp 4, a clamp 5, a Y-shaped joint 6 (a sound transmitting unit), a catheter 7 connected to a nutrition bottle, and a nutrition catheter 8 inserted in a body. The nutrition catheter 8 was inserted in the body such that a leading end portion 9 of the catheter 8 reaches inside the stomach. The operation of causing the leading end portion 9 of the nutrition catheter 8 inserted in the body to reach inside the stomach and the confirmation of the leading end portion 9 were performed as follows. First, the sound generating unit 2 was caused to produce a sound by the airflow blown therein by the syringe 1. The sound emitted from the leading end opening 9 of the catheter 8 is the loudest. Therefore, the operation of guiding the leading end portion 9 into the stomach was performed while extracorporeally detecting the emitted sound with a stethoscope. After completion of the guiding operation, to further confirm whether the leading end portion 9 has reached inside the stomach, the liquid suctioned from the catheter 8 may be checked with a litmus paper, for example, to confirm whether the liquid is acidic. A publicly known nutrition catheter can be used as the nutrition catheter 8 excluding the syringe 1 and the sound generating unit 2 (the vent member including the check valve 3).

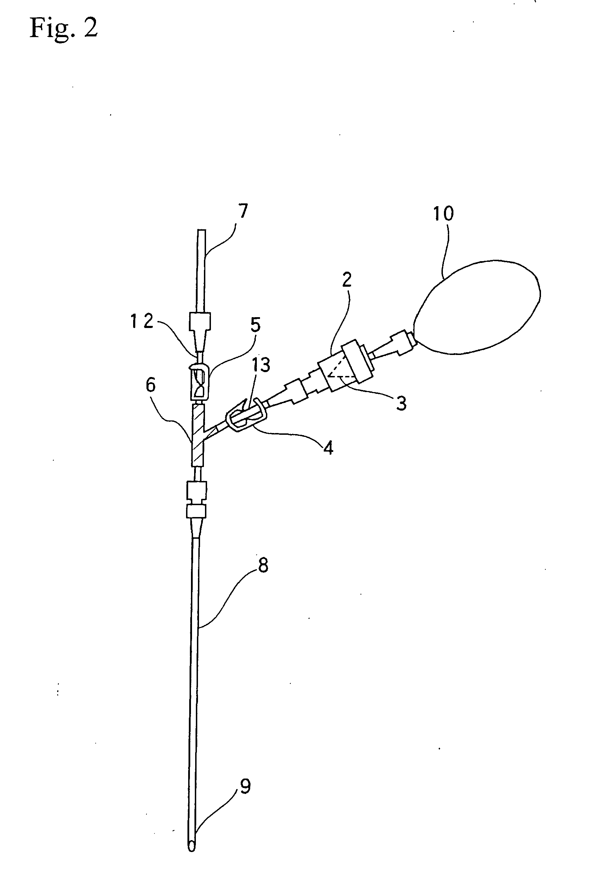

Description will be made on the basis of Fig. 2. Fig. 2 illustrates an embodiment in which a balloon 10 is used in replacement of the syringe 1, which is the airflow blowing member used in Fig. 1.

### Brief Description of the Drawings

Fig. 1 illustrates a sound producing catheter according to the present invention using a Y-shaped joint as a sound transmitting unit and a syringe as an airflow blowing member.
Fig. 2 illustrates a sound producing catheter according to the present invention using the Y-shaped joint as the sound transmitting unit and a balloon as the airflow blowing member.
Fig. 3 illustrates the sound producing catheter illustrated in Fig. 1 using a plurality of sound generating units.
Fig. 4 illustrates a sound producing catheter according to the present invention using the sound generating units and the syringe as the airflow blowing member.
Fig. 5 illustrates a sound producing catheter according to the present invention including a leading end portion formed with a small hole and a basal end provided with the syringe used as the airflow blowing member.
Fig. 6 is a perspective view of an example of a check valve used in the present invention.
Fig. 7 is a side view of the check valve illustrated in Fig. 6.

### Reference Numerals

1 syringe
2 sound generating unit
3 check valve
4 clamp
5 clamp
6 sound transmitting member (Y-shaped joint)
7 tube connected to a nutrition bottle
8 nutrition catheter inserted in a body
9 leading end portion of the catheter 8
10 balloon
11 tube having a smaller inner diameter than the inner diameters of the sound generating unit and members located at the biological body side from the sound generating unit
12 tube
13 tube
14 small hole formed at the leading end portion of the catheter
15 sloped portion of the check valve
16 insertion hole of the check valve
17 cylindrical portion at an opening of the check valve

### Best Mode for Carrying Out the Invention

As a sound producing catheter according to the present invention, a configuration example of a nutrition catheter will be described.

### EXAMPLE 1

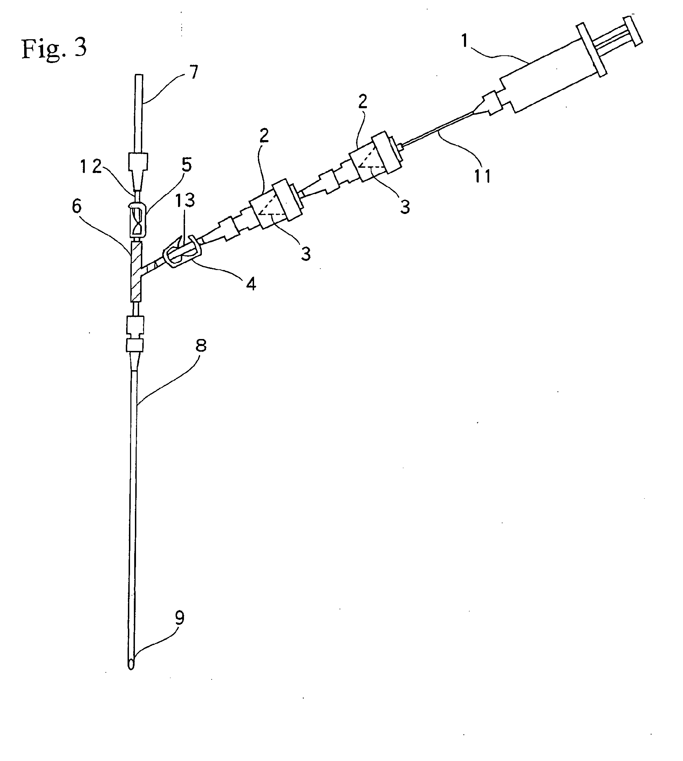

On the basis of Fig. 3, the present example will be described. One branch path of a Y-shaped joint 6 which is a sound transmitting unit is connected to a tube 13, two sound generating units (vent members each including a check valve 3) 2, and an airflow blowing member (syringe) 1. Further, one end portion of the other branch path of the Y-shaped joint 6 is provided with a nutrition catheter 8, while the other end portion is provided with a tube 12 connected to a nutrition bottle. The tubes 12 and 13 are provided with clamps 5 and 4, respectively. Further, the sound generating units 2 and the airflow blowing member (syringe) 1 are coupled to each other via the interposition of a tube 11 having a smaller inner diameter than the inner diameters of the sound generating units 2 and respective members located at the body side from the sound generating units 2. The nutrition catheter of the present example was able to reduce a sound leaking from the sound generating units, and to allow sound detecting means provided outside the body, such as a stethoscope, for example, to easily confirm the existence position of a leading end portion or the entirety of the catheter inserted in the body.

### EXAMPLE 2

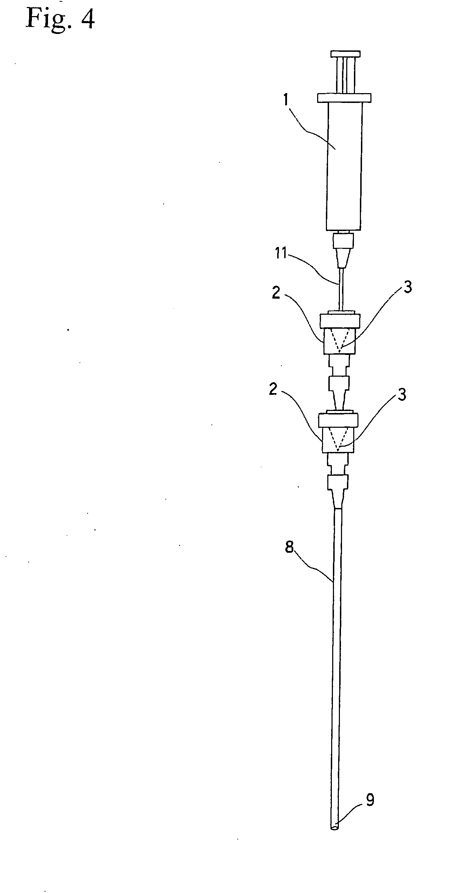

On the basis of Fig. 4, the present example will be described. Fig. 4 illustrates an end portion of the nutrition catheter 8 opposite to an end portion thereof placed in the body. The end portion of the nutrition catheter 8 was connected to the two valves 3 (the vent members), which are arranged in series. Thereby, the sound generating units are formed. Further, the vent members (or the sound generating units) and the airflow blowing member (syringe) 1 are coupled to each other via the interposition of the tube 11 having the smaller inner diameter than the inner diameters of the sound generating units 2 and the respective members located at the body side from the sound generating units 2. Similarly to the catheter according to the preceding example, the catheter according to the present example was able to reduce the sound leaking from the sound generating units 2, and to allow the sound detecting means provided outside the body, such as the stethoscope, for example, to easily confirm the existence position of the leading end portion or the entirety of the catheter inserted in the body.

### EXAMPLE 3

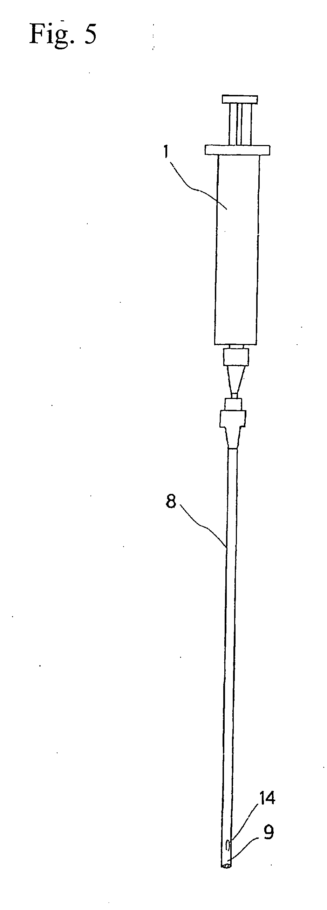

On the basis of Fig. 5, the present example will be described. Fig. 5 illustrates the end portion of the nutrition catheter 8 opposite to the end portion thereof placed in the body. The end portion of the nutrition catheter 8 was connected to the airflow blowing member (syringe) 1. The nutrition catheter 8 is formed, at a leading end side thereof placed in the body, with a small hole which is caused to produce a sound by the airflow blowing member (syringe) 1. In the sound producing catheter according to the present embodiment, the sound is produced in the body by the airflow blowing member (syringe) 1. Accordingly, the confirmation of the leading end position of the catheter by using the sound detecting means provided outside the body, such as the stethoscope, for example, can be performed more accurately than the catheters according to the above-described examples 1 and 2.

### Industrial Applicability

According to the present invention, it is possible to confirm the position of a catheter inserted in a body more easily, reliably, and safely, and also independently of the state inside the stomach of a patient. Accordingly, the present invention was able to provide a catheter which solves the problems of the above-described conventional techniques.

## Claims

1. A member for confirming the position of a catheter placed in a body, the member **characterized by** comprising a sound generating unit, and **characterized in that** a sound generated from the sound generating unit can propagate through the catheter.

2. The member for confirming the position of a catheter placed in a body as described in Claim 1, the member **characterized by** comprising a sound transmitting unit in addition to the sound generating unit, and **characterized in that** the sound generated from the sound generating unit can propagate through the catheter via the sound transmitting unit and an opening formed at a leading end side of the transmitting unit.

3. The member for confirming the position of a catheter placed in a body as described in Claim 2, the member **characterized in that** the sound transmitting unit is a straight-chain path or a branch path.

4. The member for confirming the position of a catheter placed in a body as described in any one of Claims 1 to 3, the member **characterized in that** the confirmed position of the caterer is the leading end position and/or the entire position of the catheter.

5. The member for confirming the position of a catheter placed in a body as described in any one of Claims 1 to 4, the member **characterized in that** the sound generating unit is caused to produce the sound by airflow.

6. The member for confirming the existence position of a catheter placed in a body as described in Claim 5, the member **characterized in that** the production of the sound caused by the airflow is performed by a syringe or a balloon which is an airflow blowing member.

7. A catheter **characterized by** being connected, at an end portion thereof opposite to an end portion thereof placed in a body, to the member for confirming the position of a catheter placed in a body as described in any one of Claims 1 to 6.

8. The catheter as described in Claim 7, **characterized in that** the sound generating unit forms a coupling member for coupling the catheter to the airflow blowing member.

9. The catheter as described in Claim 8, **characterized in that** the airflow blowing member and the sound generating unit are coupled to each other via the interposition of a tube having a smaller inner diameter than the inner diameters of the sound generating unit and members located at the biological body side from the sound generating unit.

10. The catheter as described in any one of Claims 7 to 9, **characterized in that** the sound generating unit includes a valve structure, and that two or more of the sound generating units are arranged in series.

11. A catheter **characterized by** comprising a sound generating unit at a leading end portion thereof placed in a body.

12. The catheter as described in Claim 11, **characterized in that** the sound generating unit is caused to produce a sound by airflow.

13. The catheter as described in Claim 12, **characterized in that** the production of the sound caused by the airflow is performed by a syringe or a balloon which is an airflow blowing member.

14. The catheter as described in any one of Claims 7 to 13, capable of confirming the leading end position of the catheter.

15. The catheter as described in Claim 14, wherein the leading end position of the catheter is confirmed when the leading end of the catheter has reached inside the stomach.

16. The catheter as described in any one of Claims 7 to 13, **characterized in that** the position of the catheter is confirmed by sound detecting means provided outside the body.

17. The catheter as described in any one of Claims 7 to 16, **characterized in that** the catheter is a nutrition catheter.

18. A method of confirming the position of a catheter, wherein the existence position in a body of the catheter as described in any one of Claims 7 to 17 is confirmed by sound detecting means provided outside the body.

19. The method of confirming the position of a catheter as described in Claim 18, wherein the position of the catheter in the body is confirmed when the leading end of the catheter has reached inside the stomach.
